# EUROPÄISCHE PATENTSCHRIFT

(11) **EP 2 631 257 B1**
(45) Veröffentlichungstag und Bekanntmachung des Hinweises auf die Patenterteilung: **20.07.2016**
(21) Anmeldenummer: 13168848.3
(22) Anmeldetag: 23.11.2010
(51) Int. Cl.: C08G 63/16, C07C 29/80, C08G 18/32

(54) **VERFAHREN ZUR HERSTELLUNG VON KUNSTSTOFFEN MIT 1,6-HEXANDIOL MIT EINEM ALDEHYDANTEIL VON KLEINER 500PPM**
METHOD FOR PRODUCING PLASTICS WITH 1,6-HEXANEDIOL WITH AN ALDEHYDE CONTENT OF LESS THAN 500PPM
PROCÉDÉ DE FABRICATION DE MATIÈRES SYNTHÉTIQUES À L'AIDE DE 1,6-HEXANDIOL AVEC UNE PART D'ALDÉHYDE INFÉRIEURE À 500 PPM

(30) Priorität: 26.11.2009 DE 102009047194
(43) Veröffentlichungstag der Anmeldung: 28.08.2013
(62) Teilanmeldung aus: 10779319.2
(73) Patentinhaber: BASF SE, 67056 Ludwigshafen am Rhein (DE)
(72) Erfinder: Pinkos, Rolf, 67098 Bad Dürkheim (DE); Kleinmann, Eva, 68163 Mannheim (DE); Abillard, Olivier, 68159 Mannheim (DE); Gehringer, Lionel, 67470 Schaffhouse-pres-Seltz (FR)
(74) Vertreter: BASF IP Association

(56) Entgegenhaltungen:
- WO-A1-97/31882
- DE-A1- 2 060 548
- DE-A1- 2 062 433

## Beschreibung

Die vorliegende Erfindung betrifft ein Verfahren zur Herstellung von Kunststoffen mit Hilfe von 1,6-Hexandiol mit einem Aldehydanteil von kleiner 500 ppm, sowie ein Verfahren zur Herstellung von 1,6-Hexandiol mit einem Aldehydanteil von kleiner 500 ppm. 1,6-Hexandiol ist ein wertvolles Zwischenprodukt zur Herstellung von Polyestern, Acrylaten oder Polyurethanen. 1,6-Hexandiol ist dabei allgemein zugänglich durch Hydrierung von Adipinsäure oder Adipinsäure-haltigen Feedströmen, die die Adipinsäure beispielsweise in Wasser oder als Ester wie Adipinsäuredimethylester oder durch Hydrierung von Hydroxycapronsäure oder deren Ester oder durch Hydrierung von Caprolacton, wie es von K. Weissermel, H.-J. Arpe et al. in Industrielle Organische Industrie, Fünfte Auflage, Wiley-VCH, Seiten 267 und 269 beschrieben, enthalten.

Das kommerziell erhältliche 1,6-Hexandiol weist, wie beispielsweise im Datenblatt der Firma Lanxess ausgewiesen, trotz hoher Reinheit von 99,8 FI.-% noch Komponenten auf, die die Anwendungsbreite einschränken können. Das 1,6-Hexandiol ist als weißer bis leicht gelblicher Feststoff bzw. ebensolche Flüssigkeit beschrieben, das bis zu 0,1 Gew.-% 6-Hydroxyhexanal enthält. Es ist allgemein bekannt, dass die Anwesenheit von Aldehyden die Farbzahlstabilität von Produkten einschränkt. Diese Aldehyde können einerseits frei vorliegen, jedoch auch als Halbacetale bzw. Acetale und haben als solche, einen ebenfalls negativen Einfluss auf die Farbzahl von Produkten, beispielsweise Polyestern. Außerdem sind solche Verbindungen auch insofern anwendungstechnisch unerwünscht, als sie kein Diol darstellen und, z.B. bei der Herstellung von Polyestern, zu Kettenabbruch bzw. zu Verzweigungen führen.

Zumeist werden Polyester und insbesondere Polyesteralkohole durch Polykondensationsreaktionen von mehrwertigen Carbonsäuren / Carbonsäurenderivaten mit mehrwertigen Alkoholen bzw. Polyolen bei Temperaturen von insbesondere 150-280°C unter Normaldruck und oder leichtem Vakuum im Beisein von Katalysatoren hergestellt.

Im vorliegenden Fall sind als unerwünschte Verunreinigungen folgende Komponenten auf Basis 6-Hydoxyhexanal bzw. 6-Hydroxyhexanal selbst relevant und sollen in der vorliegenden Erfindung unter dem Überbegriff "Aldehyd" zusammengefasst verstanden werden:

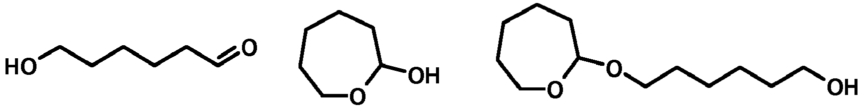

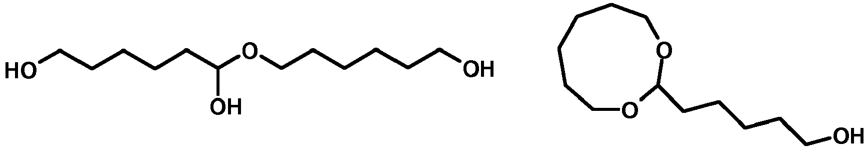

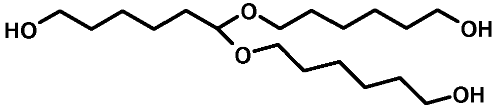

Eine weitere Komponente, die sich aus Hydroxyhexanal bilden kann und in größeren Mengen ebenfalls unerwünscht ist, ist 6-Hydroxycapronsäure-1,6-hexandiolester, im Folgenden gezeigt:

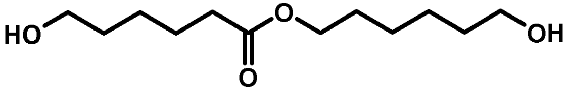

Dieser Ester kann sich unter denselben unerwünschten Bedingungen aus 1,6-Hexandiol bilden wie das 6-Hydroxyhexanal. Dieser Ester kann mit einer sog. Basenzahl erfasst werden und wird dabei durch Titration mit KOH bestimmt. Liegt eine Basenzahl beispielsweise bei 8 und wird diese allein durch den o.g. Ester verursacht, so liegt der Estergehalt bei ca. 33 ppm. Der Ester kann im Prinzip als Diol aufgefasst werden und stört bei Gehalten unter 500 ppm, insbesondere unter 50 ppm im Allgemeinen nicht bei Polyesteranwendungen und verursacht auch keine Farbe.

WO 97/31882 A1 beschreibt ein Verfahren zur Herstellung von 1,6-Hexandiol, in dem ein aus der Hydrierung eines Gemisches, welches im Wesentlichen Adipinsäure und 6-Hydroxycapronsäure enthält, resultierender Austrag bei einem Druck von 1 bis 1000 mbar destilliert wird. Gemäß dem Beispiel konnte durch Destillation bei 20 mbar eine 1,6-Hexandiol-Fraktion mit einer Reinheit von 99,8% erhalten werden.

Die Aufgabe der vorliegenden Anmeldung ist daher, ein Verfahren zur Herstellung von Kunststoffen bereit zu stellen, das es ermöglicht, diese mit Farbzahlen kleiner 150 APHA-Hazen gemäß ISO 6271 herzustellen. Eine weitere Aufgabe der vorliegenden Erfindung ist es, ein Verfahren bereit zu stellen, mit dem 1,6-Hexandiol hergestellt werden kann, das selbst eine Farbzahl kleiner 30 APHA-Hazen und gleichzeitig eine Reinheit von mehr als 97 % bei einem Aldehydgehalt von unter 500 ppm aufweist.

Diese Aufgabe wird gelöst durch ein Verfahren zur Herstellung eines Kunststoffes, der die Umsetzung von 1,6-Hexandiol in Gegenwart mindestens eines Katalysators mit Dicarbonsäuren oder Diisocyananten umfasst, wobei das 1,6-Hexandiol eines ist, das nach seiner Herstellung durch Hydrierung mindestens einer Destillation, in der das molare Verhältnis von Sauerstoff zu 1,6-Hexandiol weniger als 1:100 beträgt, unterzogen wird und während der Destillation ≤ 5 ppm an katalytisch wirkenden Komponenten enthält und einen Aldehydanteil von weniger als 500 ppm aufweist, wobei die katalytisch wirkenden Komponenten ausgewählt sind aus der Gruppe von metallischem Cu, dessen Legierungen, dessen Oxiden, dessen Halogeniden, dessen Carboxylaten und Mischungen enthaltend das reine Metall, Legierungen, Oxide und/oder Halogenide von Cu.

Ein weiterer Gegenstand der Erfindung ist ein Verfahren zur Herstellung von 1,6-Hexandiol mit einem Aldehydanteil von < 500 ppm umfassend folgende Schritte:
I) Bereitstellung einer Mischung, die 1,6-Hexandiol enthält,
II) gegebenenfalls Entfernung katalytisch wirkender Komponenten bis zu einem Restgehalt von ≤ 5 ppm
III) Destillation der aus Schritt I oder II enthaltenden Mischung, wobei das molare Verhältnis von Sauerstoff zu 1,6-Hexandiol während der Destillation weniger als 1:100 beträgt und der Gehalt an katalytisch wirkenden Komponenten während der Destillation ≤ 5 ppm beträgt
IV) Auffangen des aus Schritt III erhaltenen 1,6-Hexandiols, mit einem Aldehydgehalt von kleiner 500 ppm,
wobei die katalytisch wirksamen Komponenten ausgewählt sind aus der Gruppe von metallischem Cu, dessen Legierungen, dessen Oxiden, dessen Halogeniden, dessen Carboxylaten und Mischungen enthaltend das reine Metall, Legierungen, Oxide und/oder Halogenide von Cu.

Für das erfindungsgemäße Verfahren zur Herstellung der Kunststoffe muss 1,6-Hexandiol eingesetzt werden, das vorher möglichst unter Sauerstoffausschluss destilliert wird und während der Destillation unter 5 ppm katalytisch wirkende, insbesondere dehydrierend wirkende Komponenten enthält. Die Reinheit der erhaltenen Produkte sowie die Angaben der Aldehydanteile und der Mengen an katalytischen wirkenden Komponenten sind gaschromatographisch ermittelt worden und werden in dieser Anmeldung als Flächenprozente wiedergegeben bzw. sollen als solche verstanden werden.

Die für den Schritt I des erfindungsgemäßen Verfahrens ausgehende Mischung enthält dabei das zu gewinnende 1,6-Hexandiol bevorzugt in Mengen von ≥ 10 Gew.-%, besonders bevorzugt ≥ 30 Gew.-%, bezogen auf die Mischung des Schritt I.

Während der Destillation des 1,6-Hexandiols in Schritt III des erfindungsgemäßen Verfahrens sollte das molare Verhältnis Sauerstoff zu 1,6-Hexandiol ein Verhältnis von 1 : 100 nicht überschreiten. Bevorzugt ist ein Verhältnis von weniger als 1 : 1000, besonders bevorzugt weniger als 1 : 10000. Die Destillation kann in einer oder mehreren Destillationseinheiten durchgeführt werden. Bevorzugt ist eine Destillationseinheit. Als Kolonnen für die Destillation eignen sich alle dem Fachmann bekannten Kolonnen. Bevorzugt sind Packungskolonnen, Bodenkolonnen mit Siebböden, Kolonnen mit dualflow-Böden, Kolonnen mit Glockenböden oder mit Ventilböden ausgestattete Rektifikationskolonnen, Trennwandkolonnen oder Dünnschicht- und Fallfilmverdampfer, die bevorzugt im Vakuum betrieben werden. Es wird bevorzugt mindestens eine Destillationseinheit zu verwenden. Dies ist im Allgemeinen mindestens eine Kolonne ausgewählt aus der Gruppe von Packungskolonnen, Bodenkolonnen mit Siebböden, Kolonnen mit dual flow-Böden, Kolonnen mit Glöckenböden oder mit Ventilböden ausgestattete Rektifikationskolonnen, Trennwandkolonnen oder Dünnschicht- und Fallfilmverdampfer, die bevorzugt im Vakuum bei erhöhten Temperaturen betrieben wird. Dabei beträgt das molare Verhältnis von Sauerstoff zu 1,6-Hexandiol weniger als 1 : 100, bevorzugt weniger als 1 : 1000, besonders bevorzugt weniger als 1 : 10000. Wahlweise kann vor dem Schritt III des erfindungsgemäßen Verfahren auch noch eine ein- oder mehrstufige Verdampfung des aus Schritt I oder II enthaltenden Gemisches, das 1,6-Hexandiol enthält, durchgeführt werden.

Je tiefer der Destillationsdruck, desto mehr muss auf die Dichtigkeit der verwendeten Destillationseinrichtungen geachtet werden. Diese erhöhte Dichtigkeit bei Kolonnen kann mit Hilfe von speziellen Dichtungen ausgewählt aus der Gruppe von Schweißlippendichtungen, Dichtungen mit Kammprofilen und durch Verwendung von besonders glatten Dichtflächen wie auch durch Vermeidung einer Vielzahl von Flanschen oder Zugangsstellen in den Kolonnen wie z.B. zur Messung von Druck, Temperatur oder Schaugläser erreicht werden.
Eine weitere Möglichkeit zur Vermeidung von Sauerstoff ist, die Destillationseinheiten mit einem äußeren Mantel zu versehen, der beispielsweise mittels Stickstoff oder Argon inertisiert wird.
Eine weitere Maßnahme, um den Sauerstoffgehalt während der Destillation zu vermindern, ist das Zuschweißen von Flanschen.
Um den Sauerstoffgehalt während der Destillation bestimmen zu können, besteht eine Möglichkeit darin, das Abgas der Vakuumeinheit aufzufangen und das enthaltene Gasgemisch auf seine Zusammensetzung hin zu analysieren. Dabei wird eine Aussage über den Sauerstoffeintrag in die Kolonne am besten dadurch erhalten, dass die Kolonne unter den bevorzugten Bedingungen betrieben wird, allerdings ohne Zulauf.

Katalytisch wirkende Komponenten sind ausgewählt aus der Gruppe von metallischem Cu, dessen Legierungen, dessen Oxiden, dessen Halogeniden, dessen Carboxylaten und Mischungen enthaltend das reine Metall, Legierungen, Oxide und/oder Halogenide von Cu. Bevorzugt sind die katalytisch wirkenden Komponenten ausgewählt aus der Gruppe von metallischem Cu, dessen Legierungen, dessen Oxiden, dessen Halogeniden, und dessen Carboxylaten. Berechnet als Metall, sowohl als Einzelkomponente als auch als Mischung, sollte der Gehalt an katalytisch wirkenden Komponenten im Zustrom zur 1,6-Hexandiolkolonne und insbesondere zur 1,6-Hexandiolkolonne ≤ 5 ppm, bevorzugt ≤ 3 ppm, besonders bevorzugt ≤ 1 ppm liegen. Dabei ist es bevorzugt, eine entsprechende Menge der an Metall und/oder Mischungen von Metallen zwecks Vermeidung von Akkumulation in der 1,6-Hexandiolkolonne, gleichzeitig mit dem Sumpfstrom aus der Kolonne auszuschleusen, so dass bei einer Menge von z.B. 1 g/Stunde Metall im Zulauf, auch 1 g/Stunde Metall im Sumpfstrom der Kolonne ausgeschleust wird. Um den Einfluss von Metallspuren im Zulauf zur 1,6-Hexandiolkolonne möglichst klein zu halten, ist es bevorzugt, die Zulaufstelle möglichst auf geringer Höhe der Kolonne zu haben, d.h. also unterhalb der Mitte der Kolonne, besonders bevorzugt unterhalb des unteren Drittels der Kolonne, damit eine möglichst geringe Verweilzeit der Metallspuren in der Kolonne herrscht. Dies gilt auch für den Fall, dass Sauerstoff in die Kolonne eindringt. Idealerweise erfolgt der Zulauf in den Sumpf oder Sumpfumlauf der Kolonne. Allerdings kann die Trennaufgabe erfordern, dass der Zulauf höher, z.B. im mittleren Drittel der Kolonne angebracht sein muss. Dies entscheidet letztendlich der Grad an sonstigen Komponenten im zu destillierendem 1,6-Hexandiol wie z.B. Hochsieder wie Ether und Ester, die einen höheren Siedepunkt als 1,6-Hexandiol aufweisen. Im Gegensatz dazu sind die Ether und Ester, die einen niedrigeren Siedepunkt als 1,6-Hexandiol aufweisen und zu den sogenannten Leichtsiedern gehören, dabei ausgewählt aus der Gruppe von Pentandiole wie 1,5-Pentandiol oder Hexandiole wie 1,2- und/oder 1,4-Cyclohexandiole oder 1,5-Hexandiol. Dabei gilt, je mehr hochsiedende Nebenkomponenten enthalten sind, deren Siedepunkt unter den gegebenen Destillationsbedingungen mehr als 50 °C höher liegt als der des eigentlichen 1,6-Hexandiols, desto höher muss der Zulauf an der Kolonne angebracht sein. Wird eine Trennwandkolonne verwendet, so ist der Zulauf immer auf der Höhe der Trennwand, bevorzugt in der Höhe des mittleren Drittels der Trennwand. Das Gleiche gilt auch für den Seitenabzug, der sich ebenfalls auf der Höhe des mittleren Drittels der Trennwand befindet, bevorzugt gegenüber dem Zulauf. Dieser Seitenabzug muss jedoch nicht genau gegenüber des Zulaufs sitzen, sondern kann sich auch über oder unterhalb dieser Stelle innerhalb des mittleren Drittels der Trennwand befinden. Zur Vermeidung von nach und nach auftretenden Korrosionsstellen in der Kolonne sollte z.B. die Säurezahl (mg KOH/100g Probe) im Zulauf zur Destillation unter 10 liegen, bevorzugt unter 5, besonders bevorzugt unter 1.
Insbesondere bei der 1,6-Hexandiolherstellung wird in Gegenwart von Hydrierungskatalysatoren gearbeitet, deren Katalysatorreste auch noch im erhaltenen Endprodukt enthalten sein können. Die Entwicklung chemisch und mechanisch stabiler Katalysatoren ist zwar weit fortgeschritten, jedoch können diese Katalysatoren nicht verhindern, dass beim Anfahren oder Abstellen oder beim Spülen der Anlagenteile, in denen der Katalysator enthalten ist, Katalysatoraustragreste in das 1,6-Hexandiol mitgerissen werden. Daher ist es vorteilhaft, wenn das in dem erfindungsgemäßen Verfahren eingesetzte 1,6-Hexandiol vor der Destillation von Katalysatoraustragresten befreit wird, damit diese erst gar nicht in die Destillationskolonne eingetragen werden. Katalysatorbestandteile können heterogen aber auch homogen mit dem Produktstrom aus dem Herstellungsschritt des 1,6-Hexandiols mitgeführt werden.
Eine mögliche Maßnahme zur Reduzierung von heterogenen Katalysatorbestandteilen ist ein Filter vor der Destillation des 1,6-Hexandiols in Schritt III des erfindungsgemäßen Verfahrens. Besonders bevorzugt ist es, wenn der Filter nach der Hydrierung direkt eingesetzt wird. Die Filter sind dabei ausgewählt aus der Gruppe von Kerzenfiltern, Membranfiltern und Filterhilfsmitteln wie Aktivkohle und Kieselgur. Die Kerzen- und Membranfilter weisen dabei eine Maschenweite auf, die kleiner ist als die Katalysatoraustragspartikel, bevorzugt liegt die Maschenweite unter 0,1 mm, besonders bevorzugt kleiner 0,05 mm. Die Kerzen- und Membranfilter können dabei aus Metall oder Keramik sein, wobei das Metall des Filters keine katalytisch wirksame Oberfläche für die nachfolgende Destillation des 1,6-Hexandiols darstellen darf. Solche eingesetzten Filtereinheiten können als Querstromfiltration oder im Falle der Filterhilfsmittel als Tiefenfiltration, bei der ein Filterkuchen dafür sorgt, dass keine oder nur sehr wenig heterogene Katalysatorbestandteile in die Destillation gelangen. Wahlweise können auch die feststehenden Filtereinheiten mit den Filterhilfsmitteln kombiniert werden, wenn sie nacheinander durchgeführt werden.
Homogen gelöste Katalysatorbestandteile können durch eine chemisch induzierte Fällung oder durch Ionenaustauscher entfernt werden. Bevorzugt ist der Einsatz von Ionenaustauscher. Für das erfindungsgemäße Verfahren ist es vorteilhaft, wenn das zu destillierende 1,6-Hexandiol vor Einfuhr in die Destille einen Anteil von katalytisch wirksamen Komponenten von ≤ 5 ppm aufweist.
Es ist vorteilhaft, wenn die katalytisch wirkenden Komponenten, wie z.B. Katalysatoraustragsbestandteile, frühzeitig im Herstellungsprozess des erfindungsgemäßen 1,6-Hexandiols entfernt werden. Dies kann entweder dadurch erfolgen, das der Schritt II des erfindungsgemäßen Verfahrens vor oder nach einer ein- oder mehrstufigen Verdampfung der Mischung aus Schritt I des erfindungsgemäßen Verfahrens, das 1,6-Hexandiol enthält, erfolgt. Bevorzugt ist die Entfernung der katalytisch wirkenden Komponenten vor der ein- oder mehrstufigen Verdampfung. Der Einsatz einer ein- oder mehrstufige Verdampfung ist vorteilhaft, wenn die Herstellung des 1,6-Hexandiols mit einem Aldehydanteil von weniger als 500 ppm nach einem kontinuierlichen Verfahren erfolgen soll. Bei der Durchführung der ein- oder mehrstufigen Verdampfung ist es vorteilhaft, wenn die Verdampfung bei Drücken unterhalb von 200 mbar, bevorzugt unterhalb von 100 mbar und Temperaturen unter 230°C, vorteilhaft unterhalb von 180°C und Verweilzeiten von weniger als 60 min, bevorzugt weniger als 40 min geschehen, da ansonsten bei diesem Verdampfungsschritt bereits unerwünschte Dehydrierungsreaktionen ablaufen können.

Ein so vorbehandeltes 1,6-Hexandiol kann anschließend in einer Destillationskolonne gemäß Schritt III des erfindungsgemäßen Verfahrens bei der das Sauerstoff/Diol-Verhältnis kleiner als 1:100 ist, destilliert werden, so dass das 1,6-Hexandiol einen Aldehydanteil von kleiner 500 ppm aufweist.

Sollten bei der Herstellung von 1,6-Hexandiol trotz Einsatz von entsprechenden Filtern doch noch katalytisch wirkende Komponenten in die Destillationskolonne des Schritt III des Verfahrens gelangt sein, die mengenmäßig ausreichen, um den Aldehydanteil größer 500 ppm werden zu lassen, sollte die Kolonne gesäubert werden. Dies kann durch intensives Spülen mit z.B. Wasser und/oder Säuren erfolgen, bevorzugt durch Spülung mit HNO₃. Durch eine HNO₃-Spülung können auch Spuren von z.B. Cu und/oder Co entfernt werden. Bevorzugt ist dabei eine HNO₃-Konzentation in Wasser von 1 - 20 Gew.-%

Sofern keine Maßnahmen zur Vermeidung von Sauerstoff und/oder katalytisch wirkender Komponenten getroffen werden können bzw. diese unzureichend sind, so besteht noch prinzipiell die Möglichkeit, die Destillation von 1,6-Hexandiol bei sehr geringen Drücken durchzuführen, da dadurch die Destillationstemperaturen abgesenkt werden und chemische Reaktionen wie Oxidation und/oder Dehydrierungen langsamer ablaufen. Nachteilig daran ist allerdings, dass je geringer der Druck, desto größer wird der Aufwand bei Vakuumaggregat und Kolonne. Bei sehr geringem Druck sinkt beispielsweise der Massendurchsatz durch die Kolonne, so dass man diese mit größerem Durchmesser bauen muss, was erhebliche Zusatzkosten verursacht. Ein Verfahren, das es ermöglicht, unter einem Sauerstoff/1,6-Hexandiol-Verhältnis von kleiner als 1:100 zu arbeiten und/oder katalytische Dehydrierungen zu vermeiden sind daher vorteilhafter. Der bevorzugte Destillationsdruck in Schritt III des erfindungsgemäßen Verfahrens liegt deshalb über 25 mbar, bevorzugt über 40 mbar, besonders bevorzugt über 75 mbar. Die obere Grenze liegt bei 500 mbar, bevorzugt 300 mbar.

Gegenstand der vorliegenden Erfindung ist daher nicht nur die Verwendung von einem auf diese Weise hergestelltem 1,6-Hexandiol zur Herstellung von Polyestern und Polyurethanen, sondern auch das Verfahren zur Herstellung eines 1,6-Hexandiols, das einen Aldehydanteil von kleiner 500 ppm, bevorzugt kleiner 400 ppm, ganz besonders bevorzugt kleiner 100 ppm und insbesondere ganz besonders bevorzugt kleiner 50 ppm aufweist. Ein so hergestelltes 1,6-Hexandiol zeichnet sich dabei nicht nur durch den geringen Anteil an Aldehyd aus, sondern auch durch eine nach ISO 6271 ermittelte Farbzahl von unter 30 APHA-Hazen. Ein solches 1,6-Hexandiol führt somit bei der Umsetzung zum Beispiel mit Carbonsäuren in Gegenwart von Katalysatoren zu Polyestern, die die eine nach ISO 6271 ermittelte Farbzahl (Hazen Farbzahl) von weniger als 150 APHA-Hazen, bevorzugt weniger als 120 APHA-Hazen, ganz besonders bevorzugt weniger als 100 APHA-Hazen aufweisen.

Zur Herstellung der Polyester wird das so hergestellte 1,6-Hexandiol in Gegenwart von Carbonsäuren ausgewählt aus der Gruppe von Bernsteinsäure, Maleinsäure, Fumarsäure, Glutarsäure, Adipinsäure, Dodecandisäure, Terephthalsäure, Isophthalsäure und Phthalsäure, besonders bevorzugt sind Bernsteinsäure und Adipinsäure ausgewählt. Für die Herstellung von Polyurethane wird das 1,6-Hexandiol in Gegenwart von Isocyanaten ausgewählt aus der Gruppe von Hexamethylendiisocanat, Toluol-2,4-diisocyanat, Diphenylmethandiisocyanat, Isophorandüsocanat und 4,4'-Diisocyanatodicyclohexylmethan umgesetzt. Für die Herstellung sowohl der Polyester als auch der Polyurethane können weitere Katalysatoren eingesetzt werden. Diese sind dabei ausgewählt aus der Gruppe von Säuren, Basen, Lewissäuren und Lewisbasen.

### Beispiele:

Die Bestimmung des Aldehydgehaltes erfolgt gaschromatographisch. Hierzu wird eine Säule DB5 mit 60 m Länge, einem Innendurchmesser von 0,32 mm und einer Filmdicke von 1 µm verwendet. Zur Messung wird ein Temperaturprofil durchlaufen, bei dem beim Start eine Temperatur von 90 °C für 5 Minuten isotherm gehalten wird, anschließend eine Aufheizrate von 5 °C/Minute bis 150°C erreicht sind, eingestellt, dann wird eine Aufheizrate von 1 °C/Minute bis 160°C eingestellt, danach wird eine Aufheizrate von 5°C/Minute bis 200°C und anschließend eine Aufheizrate von 20°C/Minute bis 300°C, gefolgt von einem 20 Minuten langen isotherm Phase eingestellt. Die Injektortemperatur betrug 250°C, während die FID-Temperatur 320°C betrug. Die Angaben des Aledhydgehalts im 1,6-Hexandiol sind als GC-Flächen-% bestimmt, bevorzugt dann, wenn der Gehalt an 1,6-Hexandiol > 97 % und der Gehalt an Aldehyd < 1000 ppm beträgt.
Die nachfolgenden Beispiele sollen verdeutlichen, wie man zu einem 1,6-Hexandiol gelangt, das einen Aldehydanteil von unter 500 ppm aufweist und darüber hinaus, wie sich ein erhöhter Aldehydgehalt auswirkt.

### Vergleichsbeispiel 1:

### Herstellung Hexandiol:

Adipinsäuredimethylester wird in der Gasphase bei 60 bar und 195 - 210°C an einem Kupfer-haltigen Katalysator hydriert. Die gesammelten Austräge (ca. 36 % Methanol, ca. 67 % 1,6-Hexandiol, Rest überwiegend 6-Hydroxycapronsäureester, Hexanol sowie weitere, darunter 6-Hydroxyhexanal, unter 500 ppm Mengenanteil liegende Verbindungen und ca. 15 ppm Cu (vermutlich durch Staubmitriss) werden destillativ aufgearbeitet. Dabei wird zuerst überwiegend Methanol bei Sumpftemperaturen bis 110 °C und Drücken von 1013 mbar absolut bis 500 mbar entfernt. Der verbleibende Sumpf wird diskontinuierlich in einer Destillationskolonne (1 m Füllkörperkolonne, Rücklaufverhältnis 5, kein Luftzutritt) fraktioniert bei 50 mbar absolut und Sumpftemperaturen von ca. 180 °C fraktioniert destilliert. Nach Abtrennung von Leichtsiedern wie Restmethanol und Hexanol gewinnt man 1,6-Hexandiol in einer Destillationsausbeute von ca. 90 % mit einer Reinheit von 99,9 %. Der 6-Hydroxyhexanal-Gehalt lag bei 500 ppm.

### Herstellung Polyester:

1325,3 g Adipinsäure, 396,6 g Hexandiol-1,6 mit einem 6-Hydroxyhexanal-Gehalt von 500 ppm, 623,0 g Butandiol-1,4 und 10 ppm Zinnoctoat wurden in einen Rundkolben mit einem Volumen von 4 Litern gefüllt. Die Mischung wurde unter Rühren auf 180 °C erhitzt und 3 Stunden bei dieser Temperatur belassen. Dabei wurde das entstehende Wasser durch Destillation entfernt.
Danach wurde die Mischung auf 240 °C erwärmt und bei dieser Temperatur belassen unten ein Vakuum auf 40 mbar, bis eine Säurezahl kleiner 1 mg KOH/g erreicht war. Der entstandene flüssige Polyesteralkohol hatte folgende Kennwerte:

| | |
|---|---|
| Hydroxylzahl: | 54,1 mgKOH/g |
| Säurezahl: | 0,1 mgKOH/g |
| Viskosität: | 690 mPa.s bei 75 °C |
| Wassergehalt: | 0,01% |
| Farbzahl: | 210 APHA-Hazen |

### Beispiel 1:

### Herstellung Hexandiol:

Vergleichsbeispiel 1 wird wiederholt, mit dem Unterschied, dass der Produktstrom nach Methanolabtrennung mittels eines Dünnschichtverdampfers (Sambay) von hochsiedenden Komponenten befreit wird (50 mbar). Das nach dieser Destillation erhaltene 1,6-Hexandiol wies eine Reinheit von über 99,9 % auf, der 6-Hydroxyhexanol-Gehalt lag unter 50 ppm.

### Herstellung Polyester:

1325,3 g Adipinsäure, 396,6 g Hexanediol-1,6 mit einem 6-Hydroxyhexanal-Gehalt von kleiner 50 ppm, 623,0 g Butandiol-1,4 und 10 ppm Zinnoctoat wurden in einen Rundkolben mit einem Volumen von 4 Litern gefüllt. Die Mischung wurde unter Rühren auf 180 °C erhitzt und 3 Stunden bei dieser Temperatur belassen. Dabei wurde das entstehende Wasser durch Destillation entfernt.
Danach wurde die Mischung auf 240 °C erwärmt und bei dieser Temperatur belassen unten ein Vakuum auf 40 mbar, bis eine Säurezahl kleiner 1 mg KOH/g erreicht war. Der entstandene flüssige Polyesteralkohol hatte folgende Kennwerte:

| | |
|---|---|
| Hydroxylzahl: | 56,8 mgKOH/g |
| Säurezahl: | 0,2 mgKOH/g |
| Viskosität: | 530 mPa.s bei 75 °C |
| Wassergehalt: | 0,01% |
| Farbzahl: | 68 APHA-Hazen |

### Vergleichsbeispiel 2:

Ein Gemisch aus Adipinsäuredimethylester, 6-Hydroxycapronsäuremethylester, hergestellt analog WO97/31882, Beispiel1 (Variante A), wird wie angegeben hydriert. Direkt nach dem Anfahren wird ein Gemisch enthalten, das neben Methanol und 1,6-Hexandiol noch Katalysatorspuren enthielt, aber It. GC-Analyse kein 6-Hydroxyhexanal. Aus diesem Gemisch wurde Methanol abdestilliert. Das resultierende Roh-Hexandiol enthielt ca. 150 ppm des Cu-Katalysators als Verunreinigung. Von diesem Gemisch wurden 135 g bei 150 mbar und Sumpftemperaturen von ca. 195 °C über eine Kolonne fraktioniert destilliert, ohne dass Luft ins Destillationssystem gelangte. Es wurde eine Fraktion erhalten, die die überwiegende Menge 1,6-Hexandiol enthielt, in der allerdings neben 93,7 % 1,6-Hexandiol noch 5,6 % 6-Hydroxyhexanal vorhanden waren. Daneben fanden sich 0,2 % 1,5-Pentandiol sowie mehrere jeweils unter 1000 ppm liegende Komponenten, darunter ca. 1,4-Cyclohexandiol 500 ppm.

Nach 2 Tagen Betriebszeit der Hydrierung waren im Rohhexandiol noch 16 ppm Cu-Katalysator zu finden. In der anschließenden Destillation von Roh-Hexandiol fanden sich in der das meiste Hexandiol enthaltenden Fraktion neben 99,25 % 1,6-Hexandiol noch 2100 ppm 6-Hydroxyhexanal.

### Beispiel 2:

Vergleichsbeispiel 2 wurde wiederholt, der Hydrieraustrag wurde über einen Filter (5 µm Maschenweite) filtriert. Im Rohhexandiol fanden sich nur noch 2 ppm Cu-Katalysator. In der anschließenden Destillation von Rohhexandiol fanden sich in der das meiste Hexandiol enthaltenden Fraktion neben 99,64 % 1,6-Hexandiol nur noch 450 ppm 6-Hydroxyhexanal.

### Beispiel 3:

Beispiel 2 wurde wiederholt, jedoch war im Rohhexandiol kein Cu-Katalysator mehr nachweisbar (Nachweisgrenze 2 ppm), weil der Hydrieraustrag über einen Filter (0,5 µm Maschenweite) filtriert worden war. In der anschließenden Destillation von Roh-Hexandiol fanden sich in der das meiste Hexandiol enthaltenden Fraktion neben 99,7 % 1,6-Hexandiol nur noch 40 ppm 6-Hydroxyhexanal.

### Vergleichsbeispiel 3

Beispiel 1 wurde wiederholt mit dem Unterschied, dass etwas Leckluft während der Hexandioldestillation ins System gelangte (Molverhältnis Sauerstoff zu Hexandiol ca. 1 : 90). In der das meiste Hexandiol enthaltenden Fraktion fanden sich neben 99,3 % 1,6-Hexandiol 3000 ppm 6-Hydroxyhexanal.
Bei Reduktion des Molverhältnisses Sauerstoff zu Hexandiol auf 1 : 1000 unter sonst gleichen Bedingungen lag der 6-Hydroxyhexanalgehalt nur noch bei 350 ppm.

## Patentansprüche

1. Verfahren zur Herstellung eines Kunststoffes, der die Umsetzung von 1,6-Hexandiol in Gegenwart mindestens eines Katalysators mit Dicarbonsäuren oder Diisocyananten umfasst, wobei das 1,6-Hexandiol eines ist, das nach seiner Herstellung durch Hydrierung mindestens einer Destillation, in der das molare Verhältnis von Sauerstoff zu 1,6-Hexandiol weniger als 1:100 beträgt, unterzogen wird und während der Destillation ≤ 5 ppm an katalytisch wirkenden Komponenten enthält und einen Aldehydanteil von weniger als 500 ppm aufweist, wobei die katalytisch wirkenden Komponenten ausgewählt sind aus der Gruppe von metallischem Cu, dessen Legierungen, dessen Oxiden, dessen Halogeniden, dessen Carboxylaten und Mischungen enthaltend das reine Metall, Legierungen, Oxide und/oder Halogenide von Cu.

2. Verfahren nach Anspruch 1, wobei der Kunststoff ausgewählt ist aus der Gruppe von Polyestern und Polyurethanen.

3. Verfahren nach einem der Ansprüche 1 bis 2, wobei vor der Destillation eine ein- oder mehrstufigen Verdampfung bei Drücken ≤ 200 mbar und Temperaturen ≤ 230°C durchgeführt wird.

4. Verfahren nach einem der Ansprüche 1 bis 3, wobei die katalytisch wirkenden Komponenten ausgewählt sind aus der Gruppe von metallischem Cu, dessen Legierungen, dessen Oxiden, dessen Halogeniden, und dessen Carboxylaten.

5. Verfahren nach einem der Ansprüche 1 bis 4, wobei das 1,6-Hexandiol vor der Destillation von katalytisch wirkenden Komponenten befreit wird.

6. Verfahren nach Anspruch 5, wobei die katalytisch wirkenden Komponenten durch Filtration, Fällung und/oder Ionenaustauscher entfernt werden.

7. Verfahren nach Anspruch 5, wobei mechanische Filter mit einer Maschenweite von < 0,1 mm zur Reduzierung von heterogenen Katalysatorbestandteilen eingesetzt werden.

8. Verfahren zur Herstellung von 1,6-Hexandiol mit einem Aldehydanteil von < 500 ppm umfassend folgende Schritte:
I) Bereitstellung einer Mischung, die 1,6-Hexandiol enthält,
II) gegebenenfalls Entfernung katalytisch wirkender Komponenten bis zu einem Restgehalt von ≤ 5 ppm
III) Destillation der aus Schritt I oder II enthaltenden Mischung, wobei das Verhältnis von Sauerstoff zu 1,6-Hexandiol während der Destillation weniger als 1:100 beträgt und der Gehalt an katalytisch wirkenden Komponenten während der Destillation ≤ 5 ppm beträgt
IV) Auffangen des aus Schritt III erhaltenen 1,6-Hexandiols, mit einem Aldehydgehalt von kleiner 500 ppm,
wobei die katalytisch wirksamen Komponenten ausgewählt sind aus der Gruppe von metallischem Cu, dessen Legierungen, dessen Oxiden, dessen Halogeniden, dessen Carboxylaten und Mischungen enthaltend das reine Metall, Legierungen, Oxide und/oder Halogenide von Cu.

9. Verfahren nach Anspruch 8, wobei Schritt II vor oder nach einer ein- oder mehrstufige Verdampfung bei Drücken ≤ 200 mbar und Temperaturen ≤ 230°C durchgeführt wird.

10. Verfahren nach Anspruch 9, wobei Schritt II vor einer ein- oder mehrstufige Verdampfung bei Drücken ≤ 200 mbar und Temperaturen ≤ 230°C durchgeführt wird.

11. Verfahren nach einem der Ansprüche 8 bis 10, wobei die katalytisch wirksamen Komponenten ausgewählt sind aus der Gruppe von metallischem Cu, dessen Legierungen, dessen Oxiden, dessen Halogeniden und dessen Carboxylaten.

## Claims

1. A process for preparing a plastic, which comprises reacting 1,6-hexanediol with dicarboxylic acids or diisocyanates in the presence of at least one catalyst, where the 1,6-hexanediol is a 1,6-hexanediol which has, after its preparation by hydrogenation, been subjected to at least one distillation in which the molar ratio of oxygen to 1,6-hexanediol is less than 1:100 and during the distillation comprises ≤ 5 ppm of catalytically active components and has an aldehyde content of less than 500 ppm, wherein the catalytically active components are selected from the group consisting of metallic Cu, alloys thereof, oxides thereof, halides thereof, carboxylates thereof and mixtures consisting of the pure metal, alloys, oxides and halides of Cu.

2. The process according to claim 1, wherein the plastic is selected from the group consisting of polyesters and polyurethanes.

3. The process according to either claim 1 or 2, wherein a single-stage or multistage vaporization at pressures of ≤ 200 mbar and temperatures of ≤ 230°C is carried out before the distillation.

4. The process according to any of claims 1 to 3, wherein the catalytically active components are selected from the group consisting of metallic Cu, alloys thereof, oxides thereof, halides thereof and carboxylates thereof.

5. The process according to any of claims 1 to 4, wherein the 1,6-hexanediol is freed of catalytically active components before the distillation.

6. The process according to claim 5, wherein the catalytically active components are removed by filtration, precipitation and/or ion exchange.

7. The process according to claim 5, wherein mechanical filters having a mesh opening of < 0.1 mm are used for decreasing the heterogeneous catalyst constituents.

8. A process for preparing 1,6-hexanediol having an aldehyde content of < 500 ppm, which comprises the following steps:
I) provision of a mixture comprising 1,6-hexanediol,
II) optionally, removal of catalytically active components to a residual content of ≤ 5 ppm,
III) distillation of the mixture obtained from step I or II, where the ratio of oxygen to 1,6-hexanediol during the distillation is less than 1:100 and the content of catalytically active components during the distillation is ≤ 5 ppm,
IV) collection of the 1,6-hexanediol having an aldehyde content of less than 500 ppm obtained from step III wherein the catalytically active components are selected from the group consisting of metallic Cu, alloys thereof, oxides thereof, halides thereof, carboxylates thereof and mixtures consisting of the pure metal, alloys, oxides and halides of Cu.

9. The process according to claim 8, wherein step II is carried out before or after a single-stage or multistage vaporization at pressures of ≤ 200 mbar and temperatures of ≤ 230°C.

10. The process according to claim 9, wherein step II is carried out before a single-stage or multistage vaporization at pressures of ≤ 200 mbar and temperatures of ≤ 230°C.

11. The process according to any of claims 8 to 10, wherein the catalytically active components are selected from the group consisting of metallic Cu, alloys thereof, oxides thereof, halides thereof and carboxylates thereof.

## Revendications

1. Procédé pour la production d'une matière plastique, qui comprend la mise en réaction de 1,6-hexanediol, en présence d'au moins un catalyseur, avec des acides dicarboxyliques ou des diisocyanates, le 1,6-hexanediol étant un 1,6-hexanediol qui après sa préparation par hydrogénation est soumis à au moins une distillation, dans laquelle le rapport molaire de l'oxygène au 1,6-hexanediol est inférieur à 1:100, et pendant la distillation contient ≤ 5 ppm de composants catalytiquement actifs et présente une teneur en aldéhyde de moins de 500 ppm, dans lequel les composants catalytiquement actifs sont choisis dans le groupe constitué par Cu métallique, ses alliages, ses oxydes, ses halogénures, ses carboxylates et des mélanges contenant le métal pur, alliages, oxydes et/ou halogénures de Cu.

2. Procédé selon la revendication 1, **caractérisé en ce que** la matière plastique est choisie dans le groupe des polyesters et polyuréthanes.

3. Procédé selon la revendication 1 ou 2, dans lequel avant la distillation on effectue une évaporation en une ou plusieurs étapes sous des pressions ≤ 200 mbars et à des températures ≤ 230 °C.

4. Procédé selon l'une quelconque des revendications 1 à 3, dans lequel les composants catalytiquement actifs sont choisis dans le groupe constitué par Cu métallique, ses alliages, ses oxydes, ses halogénures et ses carboxylates.

5. Procédé selon l'une quelconque des revendications 1 à 4, dans lequel avant la distillation on libère le 1,6-hexanediol des composants catalytiquement actifs.

6. Procédé selon la revendication 5, dans lequel on sépare les composants catalytiquement actifs par filtration, précipitation et/ou au moyen d'échangeurs d'ions.

7. Procédé selon la revendication 5, dans lequel pour la diminution des composants de catalyseurs hétérogènes on utilise des filtres mécaniques ayant une largeur de maille de < 0,1 mm.

8. Procédé pour la préparation de 1,6-hexanediol ayant une teneur en aldéhyde de < 500 ppm, comprenant les étapes suivantes :
I) préparation d'un mélange qui contient du 1,6-hexanediol,
II) éventuellement élimination de composants catalytiquement actifs, jusqu'à une teneur résiduelle de ≤ 5 ppm,
III) distillation du mélange obtenu dans l'étape I ou II, le rapport de l'oxygène au 1,6-hexanediol pendant la distillation étant inférieur à 1:100, et le teneur en composants catalytiquement actifs pendant la distillation étant ≤ 5 ppm,
IV) collecte du 1,6-hexanediol obtenu dans l'étape III, ayant une teneur en aldéhyde de moins de 500 ppm,
dans lequel les composants catalytiquement actifs sont choisis dans le groupe constitué par Cu métallique, ses alliages, ses oxydes, ses halogénures, ses carboxylates et des mélanges contenant le métal pur, alliages, oxydes et/ou halogénures de Cu.

9. Procédé selon la revendication 8, dans lequel on effectue l'étape II avant ou après une évaporation en une ou plusieurs étapes sous des pressions ≤ 200 mbars et à des températures ≤ 230 °C.

10. Procédé selon la revendication 9, dans lequel on effectue l'étape II avant une évaporation en une ou plusieurs étapes sous des pressions ≤ 200 mbars et à des températures ≤ 230 °C.

11. Procédé selon l'une quelconque des revendications 8 à 10, dans lequel les composants catalytiquement actifs sont choisis dans le groupe constitué par Cu métallique, ses alliages, ses oxydes, ses halogénures et ses carboxylates.
